# FASCICULE DE BREVET EUROPEEN

(11) **EP 4 615 314 B1**
(45) Date de publication et mention de la délivrance du brevet: **07.10.2026**
(21) Numéro de dépôt: 23754295.6
(22) Date de dépôt: 08.08.2023
(51) Int. Cl.: A61B 5/0535, A61B 5/00, A61B 5/29, A61B 5/0538, A61M 60/174, A61M 60/237, A61M 60/515

(54) **SYSTEME DE MESURE D'IMPEDANCE CARDIOGRAPHIQUE POUR LA COMMANDE D'UNE POMPE CARDIAQUE.**
SYSTEM ZUR MESSUNG DER KARDIOGRAPHISCHEN IMPEDANZ FÜR DIE STEUERUNG EINER HERZPUMPE.
CARDIOGRAPHIC IMPEDANCE MEASUREMENT SYSTEM FOR CONTROLLING A HEART PUMP.

(30) Priorité: 08.09.2022 FR 2209009
(43) Date de publication de la demande: 17.09.2025
(73) Titulaire: Fineheart, 33600 Pessac (FR)
(72) Inventeur: MALDARI, Mirko, 33000 BORDEAUX (FR); GARRIGUE, Stéphane, 33130 BEGLES (FR); MASCARELL, Arnaud, 37250 MONTBAZON (FR)
(74) Mandataire: IPAZ
(86) Numéro de dépôt international: PCT/EP2023/071973
(87) Numéro de publication internationale: WO 2024/052032

(56) Documents cités:
- US-A1- 2010 069 778
- US-A1- 2010 082 099
- US-A1- 2013 066 142
- US-A1- 2018 126 172

## Description

La présente invention se rapporte à un système de mesure d'impédance cardiographique.

Elle trouve une application particulièrement intéressante dans le domaine de dispositifs implantés de dispositifs d'assistance cardiaque.

L'impédance cardiographie (IC) est une technique bien connue de l'homme du métier.

Les variations de volume du sang dans les cavités cardiaques et le système vasculaire plus exceptionnellement entraînent des variations de la bio-impédance thoracique et/ou myocardique. L'impédance cardiographie permet de mesurer ces variations et d'afficher le résultat sous forme d'une courbe en fonction du temps et du cycle cardiaque (diastole et systole).

Plus précisément, l'impédance cardiographie peut permettre la surveillance et la mesure de variations du volume d'éjection systolique ainsi que du volume de remplissage diastolique du ventricule (droit ou gauche) du patient à l'aide d'un premier dipôle, dit dipôle émetteur. Le corps du patient est alors cartographié électriquement en injectant un courant d'amplitude fixe infra-liminaire et d'une durée de 5 à 30µs. La durée de l'impulsion est variable et obéit à la loi de Lapicque, connue de l'homme du métier.

Le courant peut aussi être injecté par séries d'impulsions (de 1 à n) de mêmes caractéristiques citées ci-dessus de façon consécutive. Un deuxième dipôle, dit dipôle récepteur, est utilisé pour mesurer la tension aux bornes de la cage thoracique ; ce dipôle peut être soit fixé à l'intérieur du corps soit à la surface de la peau. Ce dipôle peut aussi être fixé à la surface du myocarde si l'on veut se focaliser sur les variations du volume sanguin au sein des cavités cardiaques pour davantage de précision. La tension corporelle ou myocardique change en fonction du flux sanguin car le sang et les tissus ont des conductivités électriques différentes.

La tension aux bornes du dipôle récepteur permet de calculer l'impédance entre les deux dipôles.

Le suivi de l'impédance permet notamment la surveillance d'une insuffisance cardiaque chez le patient car l'insuffisance cardiaque entraîne une dilatation pathologique des cavités cardiaques qui altère la fonction contractile du coeur. Ce suivi donne de premières indications sur l'état hémodynamique du patient.

On connaît le document FR3103709B1 décrivant un système implantable pour mesurer une période pré-éjectionnelle à partir de l'impédance cardiographique. Ce système comporte deux dipôles distincts pour mesurer l'impédance cardiographique.

On connaît également le document FR3103697A1 décrivant des systèmes im-plantables comprenant également deux dipôles distincts pour mesurer l'impédance cardiographique. Les systèmes implantés peuvent comprendre d'une part un défibrillateur automatique ou un enregistreur en boucle, et d'une autre part une capsule de stimulateur cardiaque sans sonde.

Le document US 2013/066142 Al concerne un dispositif comprenant une pompe d'assistance cardiaque destinée à relier le ventricule correspondant d'un cœur à l'artère correspondante, et à pomper le sang du ventricule correspondant vers l'artère correspondante. Le dispositif décrit est aussi capable de mesures d'impédance du cœur.

La présente invention a pour but un nouveau système de mesure de l'impédance cardiographique plus efficace que les systèmes de l'art antérieur.

Un autre but de l'invention est un système intégré d'assistance cardiaque.

On atteint au moins l'un des objectifs précités avec un système de mesure de Cardio-Impédance comprenant :
- au moins une première paire d'électrodes dotée d'une première électrode et d'une deuxième électrode ; la première électrode étant connectée à un générateur configuré pour générer un signal d'excitation,
- un détecteur pour détecter le signal d'excitation,
- une unité de traitement configurée pour déterminer la Cardio-Impédance à partir du signal d'excitation détecté.

Le système selon l'invention comprend en outre une pompe cardiaque implantable, et la deuxième électrode est constituée par une partie métallique de cette pompe cardiaque.

Par signal d'excitation, également désigné par signal d'activation électrique cardiaque, on entend un signal émetteur destiné par la suite à être détecté après avoir subi des perturbations.

Avec le système selon l'invention, on réalise une mesure d'impédance cardiographique en utilisant avantageusement une partie métallique d'une pompe cardiaque comme référence pour les électrodes utilisées.

De préférence, une pompe cardiaque selon l'invention est prévue pour être disposée totalement ou partiellement à l'intérieur d'un ventricule. La partie métallique est de préférence située à l'intérieur du ventricule du cœur.

En fonctionnement, on tire ainsi profit de la présence de la pompe cardiaque en intra-ventriculaire pour obtenir des mesures de grandes précisions.

Les électrodes, par exemple sous forme d'anneaux, sont prévues pour être vissées ou suturées sur une paroi du cœur, notamment sur la paroi d'un ventricule dans lequel la pompe cardiaque est installée. Mais d'autres arrangements sont possible, comme par exemple des électrodes placées sur une paroi d'un premier ventricule et une pompe cardiaque placée à l'intérieur de l'autre ventricule.

En fonctionnement, les mesures d'impédance prennent en compte efficacement la distance entre la paroi du ventricule et une référence disposée à l'intérieur du ventricule. Les mesures sont plus précises et plus sensibles aux mouvements de la paroi de par le positionnement spécifique des électrodes.

Avantageusement, pour chaque paire d'électrodes, une électrode est destinée à être positionnée à la surface du myocarde et une autre électrode à l'intérieur du ventricule (droit ou gauche).

La partie métallique de la pompe cardiaque sert ainsi de masse pour les mesures d'impédance.

La première paire d'électrodes peut être une sonde d'un pacemaker.

La mesure d'impédance cardiographique permet ainsi de gérer les vitesses de rotation d'une turbine intra-cardiaque en fonction du volume de la cavité dans laquelle est positionnée la turbine (ou pompe) et en fonction de la phase cardiaque dans laquelle la mesure d'impédance est réalisée (en diastole ou en systole).

Selon un mode de réalisation avantageux de l'invention, le générateur peut être un générateur de courant, et le détecteur peut être un détecteur de tension connecté entre la première électrode et la deuxième électrode.

Dans ce premier mode de réalisation, dit bipolaire, on peut ainsi alimenter la première électrode par un courant à amplitude constante et recueillir la tension entre cette première électrode et la partie métallique de la pompe cardiaque. La tension détectée permet de calculer l'impédance cardiographique selon les principes connus par l'homme du métier.

Selon un autre mode de réalisation avantageux de l'invention, le système peut en outre comprendre une deuxième paire d'électrodes dotée d'une troisième électrode et d'une quatrième électrode ; la quatrième électrode étant confondue avec la deuxième électrode utilisée comme électrode de référence. Selon l'invention le générateur est un générateur de courant et le détecteur est un détecteur de tension connecté entre la troisième électrode et la deuxième électrode.

L'injection de courant est réalisée via la deuxième électrode et la tension est recueillie grâce à la troisième électrode afin d'éviter des éventuelles interférences entre l'émetteur et le récepteur.

Selon un autre mode de réalisation avantageux de l'invention, le système peut en outre comprendre une deuxième paire d'électrodes dotée d'une troisième électrode et d'une quatrième électrode ; la quatrième électrode étant constituée par ladite partie métallique de la pompe cardiaque. Selon l'invention, le générateur peut alors être un générateur de tension connecté à la première paire d'électrodes et le détecteur peut être un détecteur de tension connecté à la deuxième paire d'électrodes.

En particulier, on prévoit ainsi une paire d'électrodes émettrice composée d'un pôle sur le myocardique et d'une partie métallique de la pompe, et une deuxième paire d'électrodes réceptrice composée d'un second pôle sur le myocardique et d'une partie métallique de la pompe).

En particulier, la partie métallique utilisée dans tous les modes de réalisation peut être une partie métallique non mobile de la pompe.

Dans ce deuxième mode de réalisation, dit tripolaire, on alimente la première paire avec un signal variable dans le temps à amplitude de pic constante, et on détecte une tension sur la deuxième paire ; les deux paires ayant une référence commune qui est la partie métallique de la pompe cardiaque. De préférence, le signal variable émit par la première électrode respecte la loi de Lapicque, connue à l'homme du métier afin de ne pas stimuler artificiellement le cœur du patient.

Selon un mode de réalisation de l'invention, l'unité de traitement peut être configurée pour déterminer une variation du volume du cœur ou un mouvement d'une paroi du cœur.

Le calcul de l'impédance permet d'en déduire ensuite les variations du volume du cœur, notamment par rapport à un volume de référence, et les mouvements de la paroi.

Selon un mode de réalisation préféré, pour au moins un cycle d'une activité cardiaque, l'unité de traitement est configurée pour déterminer :
- un instant t1 correspondant au début d'une dépolarisation ventriculaire d'un cycle de l'activité cardiaque, l'instant t1 étant obtenu à partir d'un électrogramme local d'activation ventriculaire
- un instant t2 correspondant à l'ouverture de la valve aortique, l'instant t2 étant obtenu par mesure d'impédance en identifiant la valeur minimale d'impédance ou le début de la positivité de la dérivée de l'impédance par rapport au temps,
- un instant t3 correspondant à la fermeture aortique, l'instant t3 étant obtenu par mesure d'impédance en identifiant un maximum dans une courbe de variation d'impédance ou par déduction à partir d'un instant t4,
- un instant t4 correspondant au début du remplissage ventriculaire, l'instant t4 étant obtenu par mesure d'impédance en identifiant le début de la négativation de la dérivée de l'impédance par rapport au temps.

Par exemple, l'instant t1 est obtenu à partir d'un électrogramme local d'activation ventriculaire détecté par le dipôle placé à la surface du myocarde en regard du ventricule intéressé (droit ou gauche).

L'instant t2 est obtenu en utilisant un système comprenant une sonde bipolaire qui peut être le dipôle disposé sur le myocarde, et une chaîne d'acquisition dotée par exemple d'un amplificateur, de filtre et d'un microprocesseur.

L'instant t3 peut être déduit de t4 en soustrayant de l'instant t4 une valeur prédéterminée ou calculée par échographie.

On peut notamment utiliser une sonde épicardique bipolaire pour générer l'électrogramme. Cette sonde permet d'écouter le rythme ventriculaire. Il peut s'agir d'une sonde de pacemaker.

Le système selon l'invention permet d'identifier des instants remarquables du rythme cardiaque.

Avantageusement, l'unité de traitement peut être configurée pour déterminer, par exemple en temps réel ou de façon régulière, des paramètres de commande de la pompe cardiaque.

Le système selon l'invention permet de fournir des informations précieuses en temps réel sur le volume du ventricule. Ces informations sont importantes car si un patient boit peu, le volume ventriculaire est diminué, la masse volémique est diminuée et le fait de réduire la vitesse rotatoire de la pompe cardiaque fait partie de la thérapie afin d'éviter des phénomènes de succion de paroi ventriculaire exacerbée et potentiellement délétères. En revanche, si l'on constate que le volume ventriculaire augmente, il peut être nécessaire d'augmenter la vitesse de la pompe cardiaque afin d'augmenter le flux de sang éjectée pendant la systole (contraction ventriculaire) et ainsi une surcharge ventriculaire pouvant induire un œdème pulmonaire.

Le système selon l'invention permet d'utiliser avantageusement les données sur la variation du volume du ventricule pour contrôler efficacement la pompe cardiaque. Il s'agit à la fois du volume instantané permettant de piloter en temps réel ou différé la pompe cardiaque, et à la fois d'un volume moyen estimé sur plusieurs cycles cardiaques.

Avantageusement, l'unité de traitement peut être configurée pour moduler, par exemple en temps réel ou de façon régulière, des cycles de fonctionnement d'un moteur de la pompe cardiaque en fonction de la variation du volume ou du mouvement de la paroi du cœur, chaque cycle comprenant au moins une vitesse de pompe basse et une vitesse de pompe haute. En d'autres termes, la mesure d'impédance cardiographique est utilisée pour piloter la pompe cardiaque.

En complément notamment de tout ce qui précède, l'unité de traitement peut être configurée pour moduler au moins l'un des paramètres de commande suivants :
- la durée de passage de la vitesse basse de la pompe à la vitesse haute de la pompe, par exemple pendant le délai électro-mécanique,
- la durée de passage de la vitesse haute de la pompe à la vitesse basse de la pompe, par exemple pendant la phase de relaxation isovolumique,
- la durée de maintien de la vitesse haute de la pompe, par exemple pendant la durée d'éjection systolique,
- la durée de maintien de la vitesse basse de la pompe, par exemple durant la phase diastolique de remplissage ventriculaire,
- la valeur de la vitesse haute de la pompe, dite vitesse systolique,
- la valeur de la vitesse basse de la pompe, dite vitesse diastolique.

On considère que les variations d'impédance sont proportionnelles à celles du volume ventriculaire. Ces variations sont utilisées comme déclencheurs de changements de vitesse de la pompe cardiaque. En temps réel, le volume augmente en diastole (remplissage ventriculaire) et diminue en systole (éjection ventriculaire).

Ainsi, les passages de vitesse diastolique (faible vitesse pendant la phase de remplissage ventriculaire) à vitesse systolique (haute vitesse pendant l'éjection ventriculaire) peuvent être pilotés par les variations de l'impédance.

Par exemple, une valeur basse de l'impédance peut correspondre à un ventricule rempli et une éjection à venir. Une valeur haute de l'impédance peut correspondre à ventricule vidé et un remplissage à venir.

La pompe cardiaque fonctionne à vitesse maximale programmée lors de l'ouverture de la valve aortique si on considère le ventricule gauche ou la valve pulmonaire si on considère le ventricule droit, et à vitesse minimale lorsque le ventricule gauche/droit se remplit de sang (phase de diastole).

On régule ainsi automatiquement le régime moteur de la pompe cardiaque en fonction du timing naturel et spontané du cœur du patient implanté.

Avec l'invention, on peut également définir des valeurs min et max ou des valeurs moyennes de l'impédance et/ou du volume du cœur. Le suivi de ces valeurs permet une gestion automatique de la vitesse d'éjection optimale de la pompe, notamment en cas de dépassement de ces valeurs.

Selon une caractéristique avantageuse de l'invention, tout ou partie des paramètres de commande peuvent être obtenu à partir des instants t1, t2, t3 et t4.

Selon un mode de mise en œuvre de l'invention, l'unité de traitement peut être configurée pour déterminer, par exemple en temps réel ou de façon régulière, un volume systolique, un volume global du ventricule, ou un indice de contractilité cardiaque en faisant un ratio ou une différence entre les valeurs maximale et minimale de la variation d'impédance pour un cycle cardiaque (diastole + systole).

On détermine un volume du ventricule gauche si le dipôle est fixé sur le myocarde du ventricule gauche ou du ventricule droit si le dipôle est fixé sur le myocarde du ventricule droit.

La valeur de contractilité cardiaque est un paramètre d'évaluation de l'efficacité non seulement de la pompe mais aussi de l'état hémodynamique et clinique du patient implanté. Le suivi de cette valeur relative mais proportionnelle à la valeur absolue de contractilité cardiaque (différence entre le volume maximal diastolique du ventricule et son volume minimal en fin de systole, i.e., en fin de contraction) au cours du temps permet de suivre l'évolution du statut hémodynamique du patient.

Une telle réalisation permet de surveiller des paramètres physiologiques et de suivre l'évolution d'une éventuelle insuffisance cardiaque.

Selon l'invention, la partie métallique peut être une partie de la pompe cardiaque destinée à être disposée soit à l'intérieur du cœur soit à l'extérieur. Cette partie métallique peut également être le caisson de la pompe cardiaque.

Selon un mode de mise en œuvre de l'invention, l'unité de traitement peut être configurée pour détecter une impédance cardiographique en réponse à une détection d'un complexe QRS d'un électrocardiogramme.

En d'autres termes, un signal issu d'un électrocardiogramme est surveillé de façon à détecter les complexes QRS. En cas de détection, on lance une procédure de mesure de l'impédance cardiographique.

Selon un autre aspect de l'invention, il est proposé un procédé de commande d'une pompe cardiaque comprenant les étapes suivantes :
a) la mesure d'impédance cardiographique en utilisant un système tel que décrit ci-dessus,
b) la variation de l'impédance cardiographique au cours d'un cycle cardiaque (systole + diastole) à partir de la variation relative de volumes ventriculaires,
c) la détermination de paramètres de commande de la pompe, et
d) l'utilisation des paramètres de commande pour piloter un moteur de la pompe cardiaque.

Avec le procédé selon l'invention, la pompe cardiaque est piloté à en fonction du volume ventriculaire, ce dernier étant obtenu par mesure d'impédance cardiographique.

Comme déjà mentionné plus haut, la détermination de paramètres de commande de la pompe peut comprendre la détermination des instants suivants :
- un instant t1 correspondant au début d'une dépolarisation ventriculaire d'un cycle de l'activité cardiaque, l'instant t1 étant obtenu à partir d'un électrogramme local d'activation ventriculaire,
- un instant t1 correspondant au début d'une dépolarisation ventriculaire d'un cycle de l'activité cardiaque, l'instant t1 étant obtenu à partir d'un électrogramme local d'activation ventriculaire,
- un instant t2 correspondant à l'ouverture de la valve aortique, l'instant t2 étant obtenu par mesure d'impédance en identifiant la valeur minimale d'impédance ou le début de la positivité de la dérivée de l'impédance par rapport au temps,
- un instant t3 correspondant à la fermeture aortique, l'instant t3 étant obtenu par mesure d'impédance en identifiant un maximum dans une courbe de variation d'impédance ou par déduction à partir d'un instant t4,
- l'instant t4 correspondant au début du remplissage ventriculaire, l'instant t4 étant obtenu par mesure d'impédance en identifiant le début de la négativation de la dérivée de l'impédance par rapport au temps.

Selon l'invention, la réalisation des étapes a) à d) peut être conditionnée à une détection d'un complexe QRS par un signal local ventriculaire d'électrogramme.

Les étapes a) à c) peuvent être réalisées sur au moins un cycle cardiaque avant la réalisation de l'étape d). De préférence, on détermine plusieurs valeurs des paramètres de commande sur les premiers cycles cardiaques, on peut moyenner les valeurs obtenues, puis on applique ces paramètres à la pompe cardiaque sur une durée ou un nombre de cycles cardiaques prédéterminés. Ensuite on détermine de nouveau les paramètres de commande avant application de ces paramètres sur une durée ou un nombre de cycles cardiaques prédéterminés, et ainsi de suite.

D'autres avantages et caractéristiques de l'invention apparaîtront à l'examen de la description détaillée d'un mode de mise en œuvre nullement limitatif, et des dessins annexés, sur lesquels :
[Fig.1] La [Fig.1] est une vue schématique d'une pompe cardiaque implanté dans un ventricule du cœur d'un patient,
[Fig.2] La [Fig.2] illustre des graphes représentatifs du volume estimé du ventricule, d'un électrocardiogramme, et d'un signal de pilotage du moteur de la pompe cardiaque,
[Fig.3] La [Fig.3] est un schéma électronique d'une configuration bipolaire pour la mesure d'impédance cardiographique selon l'invention,
[Fig.4] La [Fig.4] est une représentation d'un électrocardiogramme affichant deux cycles cardiaques,
[Fig.5] La [Fig.5] est un schéma fonctionnel illustrant différentes étapes de mises en œuvre pour une détection de signaux représentatifs de l'ouverture de la valve aortique après détection d'un complexe QRS,
[Fig.6] La [Fig.6] est un schéma électronique d'une configuration tripolaire pour la mesure d'impédance cardiographique selon l'invention,
[Fig.7] La [Fig.7] est une représentation d'un électrocardiogramme affichant cinq cycles cardiaques ainsi que des instants définissant une fenêtre de mesure,
[Fig.8] La [Fig.8] un schéma fonctionnel illustrant différentes étapes de mises en œuvre pour une détection de signaux représentatifs de l'ouverture de la valve aortique dans une fenêtre prédéfinie après détection d'un complexe QRS.

Les modes de réalisation qui seront décrits dans la suite ne sont nullement limitatifs; on pourra notamment mettre en œuvre des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à l'état de la technique antérieur. Cette sélection comprend au moins une caractéristique de préférence fonctionnelle sans détails structurels, ou avec seulement une partie des détails structurels si cette partie uniquement est suffisante pour conférer un avantage technique ou pour différencier l'invention par rapport à l'état de la technique antérieur.

Sur les figures, les éléments communs à plusieurs figures conservent la même référence.

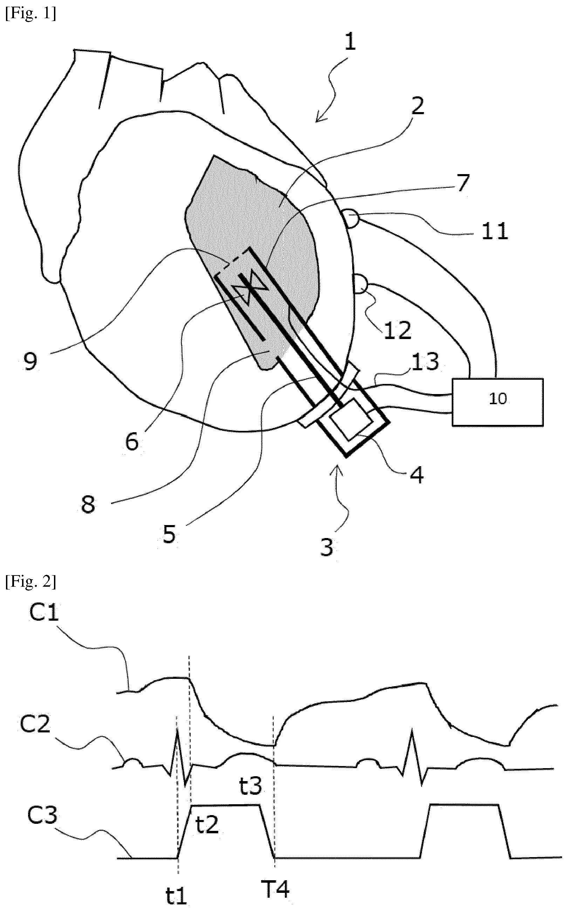

Sur la [Fig.1], on distingue un cœur 1 comprenant un ventricule 2 à l'intérieur duquel une pompe cardiaque 3 est partiellement insérée.

La pompe cardiaque 3 peut être tel que celui décrit dans le document US10,744,244. D'autres types de pompe cardiaque peuvent être utilisés, tels que les pompes drainant le sang en dérivation depuis l'apex du ventricule gauche ou droit vers l'aorte (ascendante ou descendante), qu'elles soient internalisées, externalisées, à turbine axiale/centrifuge, à membranes vibratiles/mobiles, volumétriques ou non.

Cette pompe comprend un moteur disposé à l'extérieur du cœur. Toutefois, ce moteur 4 peut être entièrement ou partiellement intégré dans le cœur ou dans le ventricule 2.

Ce moteur 4 est destiné à entraîner un arbre de transmission 5 portant des pales 6. L'ensemble arbre de transmission 5 et pales 6 a pour fonction de propulser le sang contenu dans le ventricule 2 vers l'aorte (non représenté). Pour ce faire, la pompe cardiaque 3 comprend un caisson métallique 7 autour de l'ensemble arbre de transmission 5 et pales 6, ce caisson étant doté d'une entrée 8 et d'une sortie 9. En fonctionnement, le sang contenu dans le ventricule 2 pénètre par l'entrée 8, traverse le caisson puis ressort par la sortie 9.

Idéalement, le moteur 4 fonctionne en mode pulsé, c'est-à-dire des temps de fonctionnement à vitesse élevée et des temps de fonctionnement à vitesse faible.

L'invention a pour objet de commander le moteur 4 pour que le mode pulsé corresponde au rythme cardiaque et tienne compte des caractéristiques physiologiques du cœur.

Le régime moteur est piloté par une unité de traitement 10 qui peut être implantée ou non.

Selon l'invention, l'unité de traitement 10 est reliée à une première paire d'électrodes comprenant une première électrode 11 et une deuxième électrode qui est le caisson 7 de la pompe cardiaque. L'unité de traitement 10 est donc connectée, par exemple par liaison filaire, à l'électrode 11 et au caisson 7.

Le caisson 7 sert de référence ou de masse pour tout signal électrique appliqué par l'unité de traitement 10 entre l'électrode 11 et le caisson 7.

Selon l'invention, l'unité de traitement 10 est reliée à une deuxième paire d'électrodes comprenant une troisième électrode 12 et une quatrième électrode qui est également le caisson 7 de la pompe cardiaque. La deuxième électrode et la quatrième électrode sont donc une seule et même électrode qui est le caisson 7. La première paire d'électrodes et la deuxième paire d'électrodes ont une électrode commune qui est le caisson 7.

L'unité de traitement 10 est donc connectée, par exemple par liaison filaire, à l'électrode 12 et au caisson 7.

Le caisson 7 sert également de référence ou de masse pour tout signal électrique appliqué par l'unité de traitement 10 entre l'électrode 12 et le caisson 7.

La liaison entre l'unité de traitement 10 et le caisson 7 est un fil conducteur 13.

Les électrodes 11, 12 et 7 sont utilisées pour réaliser des mesures d'impédance cardiographique. Ces mesures servent ensuite à piloter le moteur 4 pour que les pales 6 propulsent le sang depuis le ventricule 2 vers l'aorte conformément au rythme cardiaque et capacités physiologiques du patient.

Sur la [Fig.2] sont représentées trois courbes, C1 représentant le volume estimé du ventricule qui est proportionnel à l'impédance cardiographique, C2 représentant un électrocardiogramme, et C3 représentant le régime du moteur 4.

Selon l'invention, on considère les deux courbes C1 et C2. C1 est obtenue par mesure d'impédance cardiographique et C2 est obtenu à partir par exemple d'une sonde de pacemaker non représentée.

La courbe C3 est obtenu par déduction des deux courbes C1 et C2.

Pour ce faire, l'instant t1 est identifié comme étant l'instant de l'onde R d'un complexe QRS de l'électrocardiogramme (ECG). L'instant t1 correspondant au début d'une dépolarisation ventriculaire d'un cycle de l'activité cardiaque.

L'instant t2 est identifié comme étant une croissance au-delà d'un seuil prédéterminé sur la courbe C1 (moment où la dérivée de la courbe bascule de positive vers négative). L'instant t2 correspondant à l'ouverture de la valve aortique pour le ventricule gauche et la valve pulmonaire pour le ventricule droit.

L'instant t4 est identifié comme étant une croissance au-delà d'un seuil prédéterminé sur la courbe C1 (moment où la dérivée s'inverse passant de positive à négative car le ventricule se remplit de sang, début de la diastole). L'instant t4 correspondant au début du remplissage diastolique.

L'instant t3 est déterminé en déduisant une valeur prédéterminée par rapport à l'instant t4 ou lorsque la courbe d'impédance atteint une valeur minimale en plateau correspondant au début de la phase de relaxation isovolumique. L'instant t3 correspond exactement à la fermeture de la valve aortique (pour le ventricule gauche) ou la valve pulmonaire (pour le ventricule droit).

La valeur moyenne, la valeur minimale et/ou la valeur maximale du volume (courbe C1) sont utilisées pour estimer la quantité de sang à éjecter, donc la vitesse ou la puissance du moteur 4. Sur la courbe C3, le moteur de la pompe cardiaque évolue entre deux régimes, une vitesse diastolique basse à 2000 tours/minute et une vitesse systolique haute à 6000 tours/minute.

La durée entre t1 et t2 correspond au temps de montée de la pompe cardiaque entre le régime bas et le régime haut.

La durée entre t2 et t3 correspondant à la durée d'éjection (systole).

La durée entre t3 et t4 correspondant au temps de descente depuis le régime haut vers le régime bas.

La durée entre t4 et t1 correspondant à la durée diastolique.

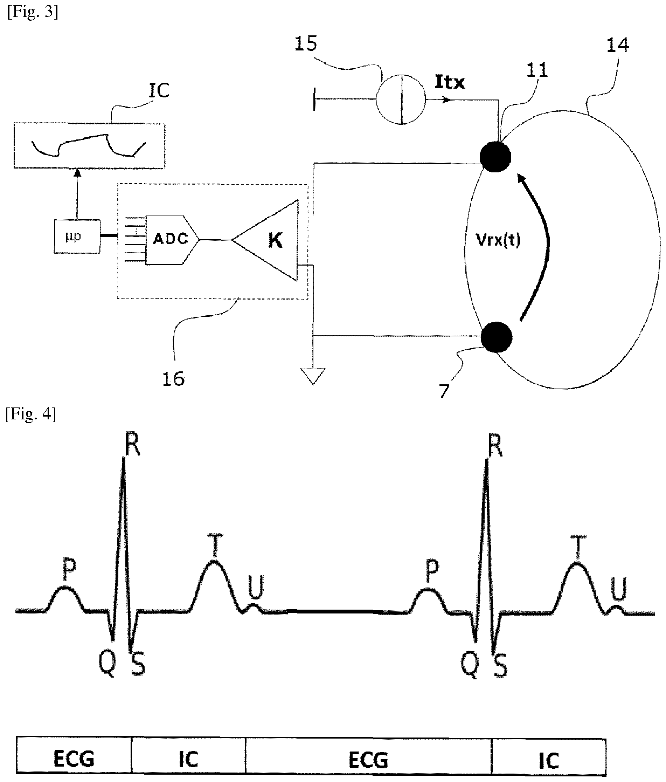

La [Fig.3] illustre un schéma électronique de mesure d'impédance selon une première configuration dite bipolaire. Dans cette configuration seule une paire d'électrodes est utilisée. On distingue la paire d'électrodes 11 et 7 telles que représentées sur la [Fig.1]. Entre les deux électrodes se trouve du tissu biologique 14 correspondant à la paroi du cœur et une partie du sang dans le ventricule 2.

L'électrode 11 est alimentée par un courant constant Itx généré par un générateur de courant 15. Ce dernier peut être intégré dans l'unité de traitement 10 ou disposé de façon indépendante.

Un détecteur 16, notamment intégré dans l'unité de traitement 10, permet de mesurer la tension Vrx entre les électrodes 11 et 7. Ce détecteur 16 comprend un amplificateur K et un convertisseur analogique numérique ADC. L'unité de traitement 10 est dotée de moyens logicielles et matériels pour traiter les signaux numériques provenant de l'ADC.

Un microprocesseur µp de l'unité de traitement traite ensuite le signal numérique de façon à calculer l'impédance cardiographique Zₜᵢₛₛᵤ en fonction du temps t par la formule suivante :
${Z}_{tissu} \left(t\right) = Vrx \left(t\right) / Itx$

La courbe C1 correspond aux différentes valeurs Ztissu(t) ou directement Vrx(t) au cours du temps. Un coefficient multiplicatif peut être appliqué entre la courbe C1 et les valeurs Ztissu(t) ou Vrx(t). Des filtres digitaux ou numériques peuvent être appliqués par le microprocesseur µp sur le signal détecté de façon à obtenir une courbe C1 facilement exploitable.

L'invention prévoit d'enregistrer les variations de cardio-impédance de façon continue ou à certaines phases spécifiques du cycle cardiaque comme il est représenté sur la [Fig.4]. Pour ce faire, on définit une seule zone temporelle entre le complexe QRS détecté et la fin de l'onde U de l'électrocardiogramme de surface dans laquelle on mesure la Cardio Impédance (CI).

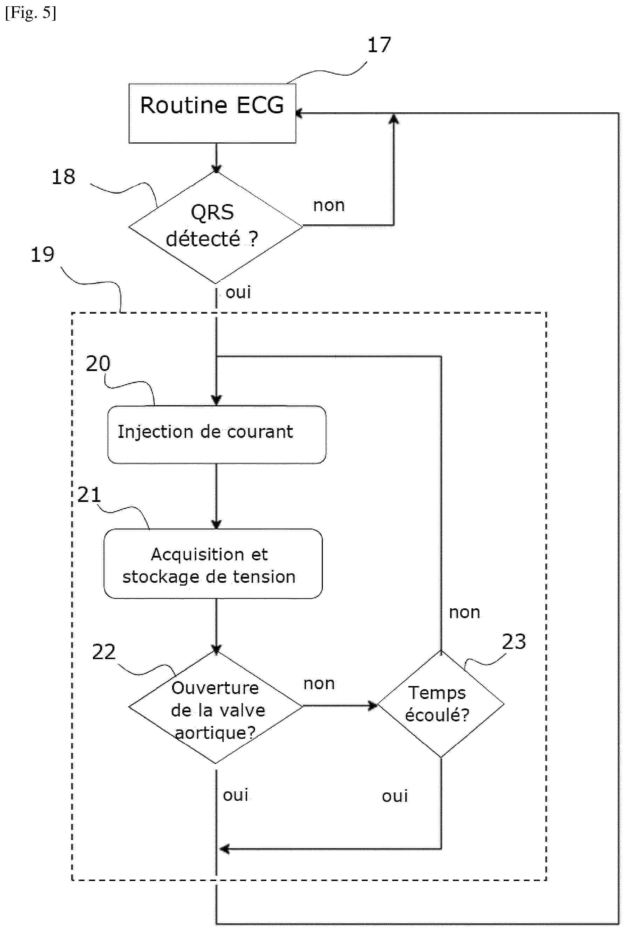

Ainsi, comme représenté sur la [Fig.5], à l'étape 17 on surveille l'électrocardiogramme, à chaque détection d'un complexe QRS à l'étape 18, on déclenche un processus 19 de mesure d'impédance. Ce processus comprend :
- une étape 20 d'injection d'un courant Itx,
- une étape 21 d'acquisition et stockage d'une tension Vrx,
- une étape 22 de vérification si la valve aortique est ouverte, pour ce faire, on calcule l'impédance cardiographique et on la compare à une valeur précédente.
- Si la valve aortique n'est pas ouverte, on vérifie à l'étape 23 si la durée de ladite première zone temporelle est écoulée. Dans l'affirmative, le processus 19 de mesure d'impédance se termine et on retourne à l'étape 17 correspondant à la deuxième zone temporelle de surveillance de l'ECG. Dans la négative, on recommence le processus de mesure d'impédance en retournant à l'étape 20 d'injection du courant.
- Si la valve aortique est ouverte, le processus 19 de mesure d'impédance se termine et on retourne à l'étape 17 correspondant à la deuxième zone temporelle de surveillance de l'ECG.

Dans tous les modes de réalisations, l'ouverture de la valve aortique correspond à détecter l'instant t2. Partant de t1, tant que l'instant t2 n'a pas été détecté, on considère que la valve aortique n'est pas encore ouverte.

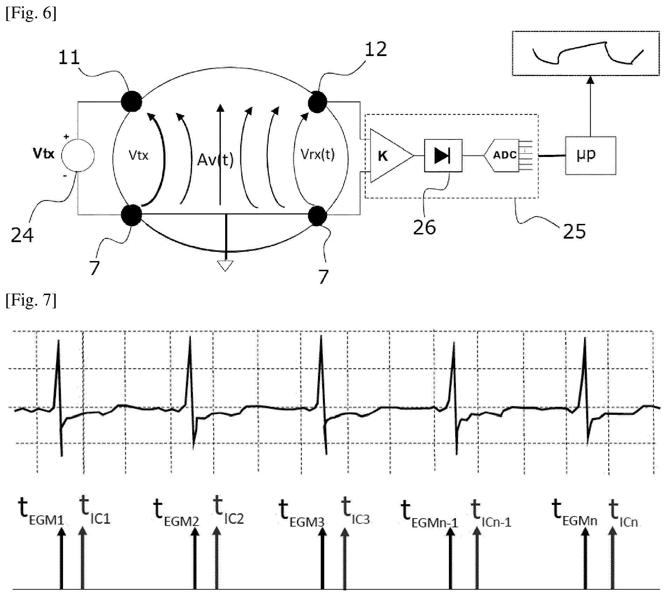

La [Fig.6] illustre un schéma électronique de mesure d'impédance selon une deuxième configuration dite tripolaire. Dans cette configuration les deux paires d'électrodes sont utilisées, la première paire en tant que dipôle émetteur, et la deuxième paire en tant que dipôle récepteur. On retrouve la paire d'électrodes 11 et 7 comme dipôle émetteur d'une part, et la paire d'électrodes 12 et 7 comme dipôle récepteur d'une autre part.

L'invention prévoit un générateur de tension 24 apte à générer une tension Vtx aux bornes de la première paire d'électrodes. Cette tension Vtx est variable dans le temps, il peut s'agir d'un signal carré ou tout autre signal à une fréquence prédéfinie f₀.

L'invention prévoit un détecteur 25, notamment intégré dans l'unité de traitement 10, pour détecter une tension Vrx due à la propagation de champ électrique créé par la tension Vtx à travers le tissu biologique. Ce champ électrique peut être représenté par une amplitude Av.

Le détecteur 25 comprend un amplificateur K, notamment doté d'un ou plusieurs filtres analogiques, connecté aux électrodes 12 et 7.

Le signal issu de l'amplificateur K peut être écrêté au moyen d'un détecteur d'enveloppe 26. Le signal écrêté est ensuite converti en un signal numérique par le convertisseur analogique-numérique ADC. Un microprocesseur µp de l'unité de traitement traite ensuite le signal numérique de façon à calculer l'impédance cardiographique par la formule :
$Vrx \left(t\right) = Vtx \left(t\right) * cos \left(2{πf}_{0}t\right) * Av \left(t\right)$

L'invention prévoit comme représentée sur la [Fig.7], un autre processus de mesure d'impédance qu'à des moments prédéterminés avec détection du moment précis de l'ouverture de la valve aortique. Cette détection précise permet de surveiller le décalage entre l'activation électrique du cœur et le l'ouverture de la valve.

Pour ce faire, on définit une zone temporelle entre l'instant T_{EGM} l'instant IC, l'intervalle de temps entre T_{EGM} et IC étant prédéfini et fixe. Dans cet intervalle de temps, on prévoit de réaliser une mesure de l'impédance cardiographique.

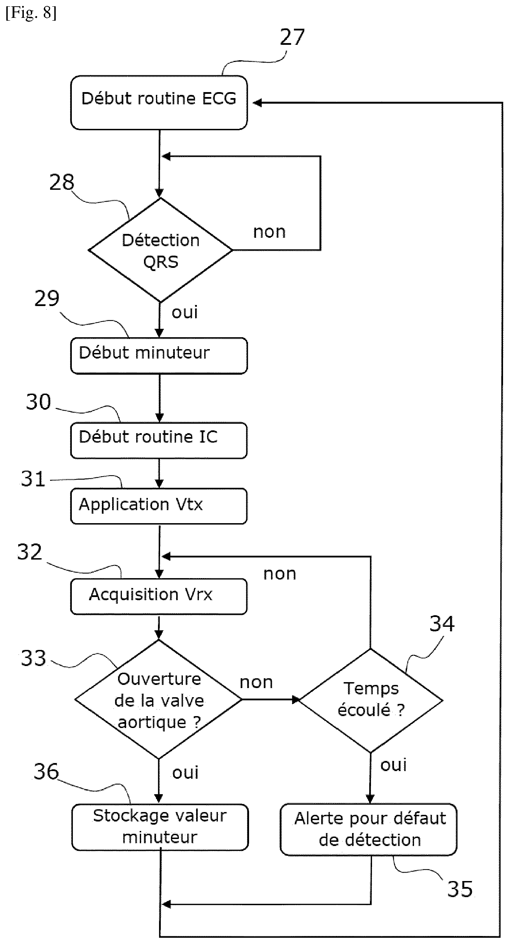

Ainsi, comme représenté sur la [Fig.8], à l'étape 27 on surveille l'électrogramme (EGM) ; à chaque détection de l'EGM ventriculaire à l'étape 28, on déclenche un minuteur à l'étape 29 et on lance le processus de mesure d'impédance à l'étape 30. Ce processus comprend :
- une étape 31 d'application d'une tension Vtx,
- une étape 32 d'acquisition et stockage d'une tension Vrx,
- une étape 33 de vérification si la valve aortique est ouverte, pour ce faire, on calcule l'impédance cardiographique et on la compare à une valeur précédente.
- Si la détection de l'ouverture de la valve aortique n'a pas eu lieu, on vérifie à l'étape 34 si la durée de ladite première zone temporelle est écoulée. Dans l'affirmative, on génère un signal d'alerte de défaut de détection à l'étape 35, puis on retourne à l'étape 27 correspondant à la deuxième zone temporelle de surveillance de l'ECG. Dans la négative, on recommence le processus de mesure d'impédance en retournant à l'étape 32 d'acquisition d'une tension Vrx.
- Si la détection de l'ouverture de la valve aortique a eu lieu, on enregistre à l'étape 36 la valeur du minuteur puis on retourne à l'étape 27 correspondant à la deuxième zone temporelle de surveillance de l'ECG.

La valeur du minuteur ainsi obtenue est à comparer à une ou d'autres valeurs précédentes pour estimer une éventuelle dégradation physiologique.

Bien entendu, l'invention n'est pas limitée aux exemples qui viennent d'être décrits.

## Revendications

1. Système de mesure de Cardio-Impédance comprenant :
- au moins une première paire d'électrodes dotée d'une première électrode (11) et d'une deuxième électrode (7) ; la première électrode (11) étant connectée à un générateur (15, 24) configuré pour générer un signal d'excitation,
- ledit générateur (15, 24),
- un détecteur (16, 25) pour détecter une tension due à la propagation de champ électrique créé par le signal d'excitation,
- une unité de traitement (10) configurée pour déterminer la Cardio-Impédance à partir de la tension détectée,
**caractérisé en ce qu'**il comprend en outre une pompe cardiaque (3) implantable et configurée pour être disposée totalement ou partiellement à l'intérieur d'un ventricule ; la deuxième électrode (7) étant constituée par une partie métallique de la pompe cardiaque (3), et **en ce que** pour chaque paire d'électrodes, la première électrode est configurée pour être positionnée à la surface extérieure d'un cœur et la deuxième électrode à l'intérieur d'un ventricule du cœur.

2. Système selon la revendication 1, **caractérisé en ce que** le générateur est un générateur de courant (15), et le détecteur est un détecteur de tension (16) connecté entre la première électrode (11) et la deuxième électrode (7).

3. Système selon la revendication 1, **caractérisé en ce qu'**il comprend en outre une deuxième paire d'électrodes dotée d'une troisième électrode (12) et d'une quatrième électrode (7); la quatrième électrode étant confondue avec la deuxième électrode utilisée comme électrode de référence ; et **en ce que** le générateur est un générateur de courant (15) et le détecteur est un détecteur de tension connecté entre la troisième électrode et la deuxième électrode.

4. Système selon la revendication 1, **caractérisé en ce qu'**il comprend en outre une deuxième paire d'électrodes dotée d'une troisième électrode (12) et d'une quatrième électrode (7) ; la quatrième électrode étant constituée par ladite partie métallique de la pompe cardiaque (3) ; et **en ce que** le générateur est un générateur de tension (24) connecté à la première paire d'électrodes et le détecteur est un détecteur de tension (25) connecté à la deuxième paire d'électrodes.

5. Système selon l'une quelconque des revendications précédentes, **caractérisé en ce que** l'unité de traitement (10) est configurée pour déterminer une variation du volume du cœur.

6. Système selon l'une quelconque des revendications précédentes, **caractérisé en ce que** l'unité de traitement (10) est configurée pour déterminer un mouvement d'une paroi du cœur.

7. Système selon l'une quelconque des revendications précédentes, **caractérisé en ce que**, pour au moins un cycle d'une activité cardiaque, l'unité de traitement est configurée pour déterminer :
- un instant t1 correspondant au début d'une dépolarisation ventriculaire d'un cycle de l'activité cardiaque, l'instant t1 étant obtenu à partir d'un électrogramme local d'activation ventriculaire
- un instant t2 correspondant à l'ouverture de la valve aortique, l'instant t2 étant obtenu par mesure d'impédance en identifiant la valeur minimale d'impédance ou le début de la positivité de la dérivée de l'impédance par rapport au temps,
- un instant t3 correspondant à la fermeture aortique, l'instant t3 étant obtenu par mesure d'impédance en identifiant un maximum dans une courbe de variation d'impédance ou par déduction à partir d'un instant t4,
- un instant t4 correspondant au début du remplissage ventriculaire, l'instant t4 étant obtenu par mesure d'impédance en identifiant le début de la négativation de la dérivée de l'impédance par rapport au temps.

8. Système selon la revendication 7, **caractérisé en ce qu'**il comprend une sonde épicardique bipolaire pour générer l'électrogramme.

9. Système selon l'une quelconque des revendications précédentes, **caractérisé en ce que** l'unité de traitement (10) est configurée pour déterminer des paramètres de commande de la pompe cardiaque (3).

10. Système selon l'une quelconque des revendications précédentes, **caractérisé en ce que** l'unité de traitement (10) est configurée pour moduler des cycles de fonctionnement d'un moteur (4) de la pompe cardiaque en fonction de la variation du volume ou du mouvement de la paroi du cœur, chaque cycle comprenant au moins une vitesse de pompe basse et une vitesse de pompe haute.

11. Système selon la revendication 10, **caractérisé en ce que** l'unité de traitement (10) est configurée pour moduler au moins l'un des paramètres de commande suivants :
- la durée de passage de la vitesse basse de la pompe à la vitesse haute de la pompe,
- la durée de passage de la vitesse haute de la pompe à la vitesse basse de la pompe,
- la durée de maintien de la vitesse haute de la pompe,
- la durée de maintien de la vitesse basse de la pompe,
- la valeur de la vitesse haute de la pompe,
- la valeur de la vitesse basse de la pompe, correspondant à une vitesse diastolique.

12. Système selon la revendication 7 et 11, **caractérisé en ce que** tout ou partie des paramètres de commande est obtenu à partir des instants t1, t2, t3 et t4.

13. Système selon l'une quelconque des revendications précédentes, **caractérisé en ce que** l'unité de traitement (10) est configurée pour déterminer un volume systolique, un volume global du ventricule, ou un indice de contractilité cardiaque en faisant un ratio ou une différence entre les valeurs maximale et minimale de la variation d'impédance pour un cycle cardiaque.

14. Système selon l'une quelconque des revendications précédentes, **caractérisé en ce que** ladite partie métallique est le caisson de la pompe cardiaque (3).

15. Système selon l'une quelconque des revendications précédentes, **caractérisé en ce que** l'unité de traitement (10) est configurée pour détecter une impédance cardiographique en réponse à une détection d'un complexe QRS d'un électrocardiogramme.

## Patentansprüche

1. Kardioimpedanzmesssystem, umfassend:
- mindestens ein erstes Elektrodenpaar, das mit einer ersten Elektrode (11) und einer zweiten Elektrode (7) ausgestattet ist; wobei die erste Elektrode (11) mit einem Generator (15, 24) verbunden ist, der konfiguriert ist, um ein Anregungssignal zu erzeugen,
- den Generator (15, 24),
- einen Detektor (16, 25) zum Erfassen einer Spannung aufgrund der Ausbreitung des durch das Anregungssignal erzeugten elektrischen Feldes,
- eine Verarbeitungseinheit (10), die konfiguriert ist, um die Kardioimpedanz aus der erfassten Spannung zu bestimmen,
**dadurch gekennzeichnet, dass** es ferner eine implantierbare Herzpumpe (3) umfasst, die konfiguriert ist, um vollständig oder teilweise im Inneren eines Ventrikels angeordnet zu werden; wobei die zweite Elektrode (7) durch ein metallisches Teil der Herzpumpe (3) gebildet wird, und dadurch, dass für jedes Elektrodenpaar die erste Elektrode konfiguriert ist, um an der äußeren Oberfläche eines Herzens positioniert zu werden, und die zweite Elektrode im Inneren eines Ventrikels des Herzens.

2. System nach Anspruch 1, **dadurch gekennzeichnet, dass** der Generator ein Stromgenerator (15) ist und der Detektor ein zwischen die erste Elektrode (11) und die zweite Elektrode (7) geschalteter Spannungsdetektor (16) ist.

3. System nach Anspruch 1, **dadurch gekennzeichnet, dass** es ferner ein zweites Elektrodenpaar umfasst, das mit einer dritten Elektrode (12) und einer vierten Elektrode (7) ausgestattet ist; wobei die vierte Elektrode mit der als Referenzelektrode verwendeten zweiten Elektrode zusammenfällt; und dadurch, dass der Generator ein Stromgenerator (15) ist und der Detektor ein Spannungsdetektor ist, der zwischen die dritte Elektrode und die zweite Elektrode geschaltet ist.

4. System nach Anspruch 1, **dadurch gekennzeichnet, dass** es ferner ein zweites Elektrodenpaar umfasst, das mit einer dritten Elektrode (12) und einer vierten Elektrode (7) ausgestattet ist; wobei die vierte Elektrode durch das metallische Teil der Herzpumpe (3) gebildet wird; und dadurch, dass der Generator ein Spannungsgenerator (24) ist, der mit dem ersten Elektrodenpaar verbunden ist, und der Detektor ein Spannungsdetektor (25) ist, der mit dem zweiten Elektrodenpaar verbunden ist.

5. System nach einem der vorstehenden Ansprüche, **dadurch gekennzeichnet, dass** die Verarbeitungseinheit (10) konfiguriert ist, um eine Änderung des Herzvolumens zu bestimmen.

6. System nach einem der vorstehenden Ansprüche, **dadurch gekennzeichnet, dass** die Verarbeitungseinheit (10) konfiguriert ist, um eine Bewegung einer Herzwand zu bestimmen.

7. System nach einem der vorstehenden Ansprüche, **dadurch gekennzeichnet, dass** die Verarbeitungseinheit für mindestens einen Zyklus einer Herzaktivität konfiguriert ist, um Folgendes zu bestimmen:
- einen Zeitpunkt t1, der dem Beginn einer ventrikulären Depolarisation eines Zyklus der Herzaktivität entspricht, wobei der Zeitpunkt t1 aus einem lokalen Elektrogramm der ventrikulären Aktivierung erhalten wird
- einen Zeitpunkt t2, welcher der Öffnung der Aortenklappe entspricht, wobei der Zeitpunkt t2 durch Impedanzmessung erhalten wird, indem der minimale Impedanzwert oder der Beginn der Positivität der Ableitung der Impedanz relativ zu der Zeit identifiziert wird,
- einen Zeitpunkt t3, der dem Aortenverschluss entspricht, wobei der Zeitpunkt t3 durch Impedanzmessung erhalten wird, indem ein Maximum in einer Impedanzänderungskurve oder durch Ableitung aus einem Zeitpunkt t4 identifiziert wird,
- einen Zeitpunkt t4, der dem Beginn der ventrikulären Füllung entspricht, wobei der Zeitpunkt t4 durch Impedanzmessung erhalten wird, indem der Beginn der Negativierung der Ableitung der Impedanz relativ zu der Zeit identifiziert wird.

8. System nach Anspruch 7, **dadurch gekennzeichnet, dass** es eine bipolare epikardiale Sonde zum Erzeugen des Elektrogramms umfasst.

9. System nach einem der vorstehenden Ansprüche, **dadurch gekennzeichnet, dass** die Verarbeitungseinheit (10) konfiguriert ist, um Steuerparameter der Herzpumpe (3) zu bestimmen.

10. System nach einem der vorstehenden Ansprüche, **dadurch gekennzeichnet, dass** die Verarbeitungseinheit (10) konfiguriert ist, um Betriebszyklen eines Motors (4) der Herzpumpe in Abhängigkeit von der Volumenänderung oder der Bewegung der Herzwand zu modulieren, wobei jeder Zyklus mindestens eine niedrige Pumpengeschwindigkeit und eine hohe Pumpengeschwindigkeit umfasst.

11. System nach Anspruch 10, **dadurch gekennzeichnet, dass** die Verarbeitungseinheit (10) konfiguriert ist, um mindestens den einen der folgenden Steuerungsparameter zu modulieren:
- die Dauer des Übergangs von der niedrigen Pumpengeschwindigkeit zu der hohen Pumpengeschwindigkeit,
- die Dauer des Übergangs von der hohen Pumpengeschwindigkeit zu der niedrigen Pumpengeschwindigkeit,
- die Dauer der Beibehaltung der hohen Pumpengeschwindigkeit,
- die Dauer der Beibehaltung der niedrigen Pumpengeschwindigkeit,
- den Wert der hohen Pumpengeschwindigkeit,
- den Wert der niedrigen Pumpengeschwindigkeit, der einer diastolischen Geschwindigkeit entspricht.

12. System nach Anspruch 7 und 11, **dadurch gekennzeichnet, dass** alle oder ein Teil der Steuerungsparameter aus den Zeitpunkten t1, t2, t3 und t4 erhalten werden.

13. System nach einem der vorstehenden Ansprüche, **dadurch gekennzeichnet, dass** die Verarbeitungseinheit (10) konfiguriert ist, um ein Schlagvolumen, ein globales Ventrikelvolumen oder einen kardialen Kontraktilitätsindex durch Bilden eines Verhältnisses oder einer Differenz zwischen den maximalen und minimalen Werten der Impedanzänderung für einen Herzzyklus zu bestimmen.

14. System nach einem der vorstehenden Ansprüche, **dadurch gekennzeichnet, dass** das genannte Metallteil das Gehäuse der Herzpumpe (3) ist.

15. System nach einem der vorstehenden Ansprüche, **dadurch gekennzeichnet, dass** die Verarbeitungseinheit (10) konfiguriert ist, um als Reaktion auf eine Detektion eines QRS-Komplexes eines Elektrokardiogramms eine kardiographische Impedanz zu erfassen.

## Claims

1. Cardio-impedance measurement system comprising:
- at least one first pair of electrodes having a first electrode (11) and a second electrode (7); the first electrode (11) being connected to a generator (15, 24) configured to generate an excitation signal,
- said generator (15, 24),
- a detector (16, 25) for detecting a voltage resulting from the propagation of the electric field created by the excitation signal,
- a processing unit (10) configured to determine cardio-impedance from the detected voltage,
**characterized in that** it further comprises an implantable cardiac pump (3) configured to be arranged wholly or partially within a ventricle; the second electrode (7) consisting of a metal part of the cardiac pump (3), and **in that** for each pair of electrodes, the first electrode is configured to be positioned on the outer surface of a heart and the second electrode within a ventricle of the heart.

2. System according to claim 1, **characterized in that** the generator is a current generator (15), and the detector is a voltage detector (16) connected between the first electrode (11) and the second electrode (7).

3. System according to claim 1, **characterized in that** it further comprises a second pair of electrodes having a third electrode (12) and a fourth electrode (7); the fourth electrode being the same as the second electrode used as a reference electrode; and **in that** the generator is a current generator (15) and the detector is a voltage detector connected between the third electrode and the second electrode.

4. System according to claim 1, **characterized in that** it further comprises a second pair of electrodes having a third electrode (12) and a fourth electrode (7); the fourth electrode consisting of said metal part of the cardiac pump (3); and **in that** the generator is a voltage generator (24) connected to the first pair of electrodes and the detector is a voltage detector (25) connected to the second pair of electrodes.

5. System according to any of the preceding claims, **characterized in that** the processing unit (10) is configured to determine a change in the volume of the heart.

6. System according to any of the preceding claims, **characterized in that** the processing unit (10) is configured to determine a movement of a wall of the heart.

7. System according to any of the preceding claims, **characterized in that**, for at least one cycle of a cardiac activity, the processing unit is configured to determine:
- a time t1 corresponding to the onset of ventricular depolarization in a cycle of the cardiac activity, time t1 being obtained from a local ventricular activation electrogram
- a time t2 corresponding to the opening of the aortic valve, time t2 being obtained by measuring impedance by identifying the minimum impedance value or the onset of the positive slope of the derivative of impedance with respect to time,
- a time t3 corresponding to aortic closure, time t3 being obtained by measuring impedance by identifying a maximum in an impedance variation curve or by deducing it from a time t4,
- a time t4 corresponding to the onset of ventricular filling, time t4 being obtained by measuring impedance by identifying the onset of the negative slope of the derivative of impedance with respect to time.

8. System according to claim 7, **characterized in that** it comprises a bipolar epicardial probe for generating the electrogram.

9. System according to any of the preceding claims, **characterized in that** the processing unit (10) is configured to determine control parameters of the cardiac pump (3).

10. System according to any of the preceding claims, **characterized in that** the processing unit (10) is configured to modulate operating cycles of a motor (4) of the cardiac pump on the basis of changes in the volume or movement of the wall of the heart, each cycle comprising at least a low pump speed and a high pump speed.

11. System according to claim 10, **characterized in that** the processing unit (10) is configured to modulate at least one of the following control parameters:
- the time it takes to switch from the low speed of the pump to the high speed of the pump,
- the time it takes to switch from the high speed of the pump to the low speed of the pump,
- the duration for which the pump maintains its high speed,
- the duration for which the pump maintains its low speed,
- the value of the high speed of the pump,
- the value of the low speed of the pump, corresponding to a diastolic speed.

12. System according to claim 7 and 11, **characterized in that** all or part of the control parameters are obtained from the times t1, t2, t3, and t4.

13. System according to any of the preceding claims, **characterized in that** the processing unit (10) is configured to determine a stroke volume, a total ventricular volume, or a cardiac contractility index by calculating a ratio or a difference between the maximum and minimum values of the impedance variation over a cardiac cycle.

14. System according to any of the preceding claims, **characterized in that** said metal part is the housing of the cardiac pump (3).

15. System according to any of the preceding claims, **characterized in that** the processing unit (10) is configured to detect a cardiographic impedance in response to the detection of a QRS complex in an electrocardiogram.
